# EUROPÄISCHE PATENTSCHRIFT

(11) **EP 1 638 678 B1**
(45) Veröffentlichungstag und Bekanntmachung des Hinweises auf die Patenterteilung: **28.09.2011**
(21) Anmeldenummer: 04739366.5
(22) Anmeldetag: 26.05.2004
(51) Int. Cl.: B01J 2/16, C12N 9/98, A23P 1/02, A61K 9/16, A23K 1/165

(54) **ENZYM-GRANULAT-HERSTELLUNGSVERFAHREN UND ERHÄLTLICHE ENZYM-GRANULATE**
METHOD FOR PRODUCTION OF ENZYME GRANULES AND ENZYME GRANULES PRODUCED THUS
PROCEDE DE FABRICATION DE GRANULES D'ENZYMES ET GRANULES D'ENZYMES AINSI OBTENUS

(30) Priorität: 11.06.2003 DE 10326231; 09.12.2003 DE 10357827; 27.01.2004 DE 102004004202; 19.02.2004 DE 102004008020
(43) Veröffentlichungstag der Anmeldung: 29.03.2006
(73) Patentinhaber: Glatt Ingenieurtechnik GmbH, D-99427 Weimar (DE)
(72) Erfinder: RÜMPLER, Karlheinz, 99425 Weimar (DE); JACOB, Michael, 99427 Weimar (DE); WASKOW, Mike, 99423 Weimar (DE)
(74) Vertreter: Maucher, Wolfgang
(86) Internationale Anmeldenummer: PCT/EP2004/005662
(87) Internationale Veröffentlichungsnummer: WO 2004/108911

(56) Entgegenhaltungen:
- EP-A- 0 332 929
- EP-A- 1 126 017
- WO-A-01/37980
- WO-A-01/83727
- DE-A- 3 609 133
- DE-A- 10 146 778
- US-A- 4 876 198
- US-A- 4 946 654

## Beschreibung

Die Erfindung betrifft ein Verfahren zur Herstellung von Enzym-Granulaten mit den im Oberbegriff des Patentanspruches 1 genannten Merkmalen, und beschreibt ein damit erhältliches Enzym-Granulat sowie dessen Verwendung zur Herstellung diese Enzym-Granulate beinhaltender Formulierungen (welche Verwendung zugleich Teil einer möglichen bevorzugten Variante des Herstellungsverfahrens für die Enzym-Granulate ist), wie in der nachfolgenden Beschreibung und den nachfolgenden Ansprüchen dargelegt.

Enzyme werden in einer Vielzahl von Industriezweigen, in immer größerem Umfang genutzt. Das betrifft sowohl die hergestellten Mengen als auch die verschiedensten Formen der Enzyme. In der Regel liegen Enzyme in flüssiger Form oder auch als Trockensubstanz vor. In der letzten Zeit werden Granulate als Handelsform von Anwendern oder von der weiterverarbeitenden Industrie immer mehr bevorzugt. Die Granulate zeichnen sich durch vorteilhafte Eigenschaften wie beispielsweise leichte Dosierbarkeit, sehr gute Fließeigenschaften, homogene innere Struktur, hohe Partikeldichte, geringen Staubgehalt sowie eine gleichmäßige und geschlossene Oberfläche aus. Da Enzyme in der Regel durch ihre besonderen Eigenschaften wie Instabilität, beispielsweise in wässriger Umgebung, und Verursachung von allergenen Reaktionen charakterisiert werden können, erweist sich die Granulatform als vorteilhafte Handelsform.

Die Stabilität von Enzymen kann dadurch verbessert werden, dass diese in eine trockene Form überführt werden. Dies kann beispielsweise durch Sprühtrocknung, verschiedene Agglomerationsprozesse (Nassgranulation in Mischern bzw. Wirbelschichtagglomeration) oder durch Aufbaugranulation in Wirbelschichtapparaten (Sprühgranulation) erfolgen.

Nachteilig bei der Sprühtrocknung ist, dass sehr große Apparatevolumina benötigt werden und das pulverförmige Produkt einen beträchtlichen Staubanteil enthält.

Um diesen Staubanteil zu verringern, wird die Sprühtrocknung häufig mittels mehrstufiger Trocknungsanlagen ausgeführt. Nachteilig ist, dass mit einer solchen mehrstufigen Trocknungsanlage hergestellte Enzym-Granulate einen schlechten, d.h. hohen Rundheitsfaktor (gibt das Verhältnis der Oberfläche eines Granulum zu der Oberfläche eines perfekt runden Granulum an) von mehr als 1,6 haben. Enzym-Granulate mit einem Rundheitsfaktor von größer als 1,6 führen, wegen der geringen Rundheit und deshalb vorhandener leicht abbrechender vorstehender Abschnitte, rasch zu einem hohen Staubanteil bei mechanischer Beanspruchung, wie sie beispielweise beim Verpacken und beim Transport auftritt.

Dieser Staubanteil erfordert spezielle Schutzmaßnahmen für das Produktionspersonal und Anwender sowie einen deutlichen Mehraufwand an Anlagentechnik zur Entstaubung, Entlüftung und zur Wiederverwertung der Stäube.

Ein mögliches Verfahren zur Herstellung von Enzym-Granulaten stellt die Aufbaugranulation in der Wirbelschicht dar, wie es in WO 01/83727 A2 veröffentlicht wurde. Hier wird ein Prozess dargestellt, bei dem die flüssige Enzym-Formulierung in eine Wirbelschicht über Sprühdüsen eingedüst wird. Der im Prozess entstehende Staub wird von der Abluft getrennt und dem Granulationsprozess als Keim wieder zugeführt. Die entstehenden Granulate werden unter Verwendung eines oder mehrerer im Anströmboden des Wirbelschichtapparates angebrachter Schwerkraftsichter aus dem Prozess entnommen. Die Größe der ausgetragenen Granulate kann durch Einstellung der Sichtgasmenge eingestellt werden. Optional können die Granulate auch zusätzlich beschichtet werden. Das Verfahren wendet den Wirbelschicht-Prozess gemäß EP-A-0163836 und EP-A-0332929 an.

Der beschriebene Wirbelschichtprozess zeichnet sich dadurch aus, dass zur gleichmäßigen Verteilung des zur Fluidisation und Trocknung benötigten Prozessgases ein Anströmboden über den gesamten Querschnitt des Wirbelschichtapparates angebracht ist. Die zum Einbringen der Flüssigkeit genutzten Sprühdüsen sprühen vertikal nach oben und sind direkt im Anströmboden integriert (EP-A-0332929) oder werden in Höhe des Anströmbodens von einem Sichter umgeben (EP-A-0163836). Die für den Prozess benötigten Granulationskeime werden durch teilweise Sprühtrocknung der eingedüsten Flüssigkeit durch teilweise Nichtüberdeckung (Hindurchsprühen) der Sprühdüsen mit dem Wirbelschichtmaterial produziert. Die Wirbelschichtmasse wird durch einen Gleichgewichtszustand zwischen sprühgetrockneten Keimen und durch den Sichtvorgang zurückgeführtem Unterkorn sowie dem Granulataustrag gebildet. Eine Abtrennung von zu großen Granulaten gibt es nicht.

Bedingt durch das Einbringen der Flüssigkeit werden die in der Wirbelschicht enthaltenen Teilchen im bedüsten Bereich mit der Flüssigkeit benetzt und es findet eine Trocknung des Flüssigkeitsfilmes auf der Teilchenoberfläche statt. Im restlichen Bereich der Wirbelschicht findet außerhalb der Düsen keine Trocknung von im Wesentlichen oberflächlich befeuchteten Teilchen statt. Statt dessen wird nur ein geringer Anteil an in den Poren der Teilchen enthaltener Feuchtigkeit verdampft, was zu einem Ansteigen der (mittleren) Partikeltemperatur führt. Eine Zuführung von erhitzten Prozessgasen ist jedoch auch außerhalb des Sprühbereiches der Düsen in herkömmlichen Wirbelschichten notwendig, um die Partikeln im Apparat zu durchmischen und ständig Teilchen in den Bedüsungsbereich zu befördern. Da die Herstellung von Enzymen temperatursensibel ist, kann mit diesen bekannten Verfahren keine optimale Ausbeute an Aktivität von Enzymen (geringe relative Aktivität bezogen auf die ursprünglich eingesetzte Enzymaktivität, d.h., neben aktivem Enzym liegt ein zu großer Anteil an inaktiviertem oder zerstörtem Enzym vor, was bedeutet, dass für dieselbe Menge an Gesamtaktivität [absolute Aktivität] mehr Enzym eingesetzt werden muss) erzielt werden. Außerdem können Temperaturungleichverteilungen im Herstellungsprozess nicht vermieden werden.

Bei dieser Prozessführung in den beschriebenen Systemen kann die Verweilzeit nur dadurch vermindert werden, dass die Trocknung der Granulate nicht bis zum erforderlichen Endwert erfolgt und/oder ein Enzym-Granulat geringerer Korngröße hergestellt wird, was jedoch die Qualität des Enzym-Granulates beeinträchtigt. Die nach dem Stand der Technik bekannten Enzym-Granulate haben einen hohen Anteil an inaktivem Trägermaterial und somit eine geringe absolute Aktivität, einen hohen Anteil an inaktiviertem Enzym (geringe relative Aktivität), einen niedrigen Wert der mittleren Korngröße D50 (Korngröße, bei der 50 Gew.-% der Teilchen einen Durchmesser kleiner und 50 Gew.-% der Teilchen einen Durchmesser größer als die mittlere Korngröße D50 haben) oder einen hohen Feuchtigkeitsgehalt, oder meist zwei oder mehr dieser Eigenschaften.

Beispielsweise kann nach einem in der WO 01/83727 A2 beschriebenen Verfahren eine Ausbeute an Enzymaktivität von mehr als 85 % (bezogen auf die theoretisch mögliche Gesamtenzymaktivität) nur bei kleinen Partikeln und/oder einem Feuchtigkeitsgehalt (Restfeuchte) von mehr als 5 % erreicht werden.

Die WO 98/55599 A2 andererseits beschreibt ein Verfahren zur Herstellung von Enzym-Granulaten unter Verwendung eines Extrusionsgerätes und eines Rondierapparates bei Verwendung eines Trägermaterials (wie Maisstärke). Dieses Verfahren ist auch beschrieben in Beispiel 2 der WO 01/83727.

Hierbei wird eine Enzymaktivitätsausbeute von 95 % (relative Enzymaktivität) erzielt und ein Granulat mit einer mittleren Korngröße D50 von 600 µm, einem Feuchtegehalt von 5 % und einem Rundheitsfaktor von 1,4. Dieses Verfahren weist den Nachteil auf, dass einem Enzympräparat mit 27 % Trockensubstanz Stärke in einem Gewichtsverhältnis von 1 : 2 beigemischt werden muss, um eine extrudierbare Mischung zu erreichen. Das durch das Extrusionsverfahren gewonnene Enzym-Granulat weist dadurch einen Gehalt an aktivem Enzymmaterial von weniger als 13 % (absolute Enzymaktivität) auf, bezogen auf die Trockensubstanz.

Das mit dem Sprühtrocknungsverfahren nach WO 01/83727 erzielbare Enzym-Granulat ergibt zwar Granulat mit einem Rundheitsfaktor im bevorzugten Bereich von 1 bis 1,6 und ebenso mit Partikeln einer mittleren Korngröße D50 von 620 µm (siehe Tabelle 2 Experiment 2), jedoch liegt der Gehalt an inaktivem Trägermaterial viel niedriger, wodurch der Gehalt an Gesamt-Enzym (aktiv und inaktiviert) höher liegt als bei dem in WO 98/55599 beschriebenen Verfahrensprodukt. Nachteilig ist jedoch bei dem Enzym-Granulat nach WO 98/55599, wie auch aus dem genannten Beispiel 2 in WO 01/83727 hervorgeht, dass der relative Anteil an aktivem Enzym, bezogen auf die Gesamtmenge aus aktivem und inaktivem Enzym, mit 85 % deutlich niedriger ist als beim Extrusionsverfahren.

Nach der in WO 01/83727 beschriebenen Arbeitsweise werden die Enzym-Granulate nach dem Verfahren gemäß EP 0 332 929 hergestellt. Dieses Verfahren hat die Eigenschaft, dass der Bettinhalt sich selbständig einstellt (siehe EP 0332 929, S. 22, Zeile 27) Dadurch ist für eine bestimmte Granulierungsleistung die Verweilzeit nicht mehr kontrollierbar. So ist in Beispiel 1 der Inhalt des Wirbelbetts 3 kg und die Granulierungsleistung liegt bei 1,5 kg/Stunde bei Granulierung aus einer wässrigen Kochsalzlösung mit einem Gehalt von 23 Gew.-% Trockenstoff. Die Verweilzeit ist also in diesem Falle fixiert auf 2 Stunden. Somit ist die Verweilzeit dort festgelegt durch das Verhältnis von Bettinhalt zu Granulierungsleistung in kg/Stunde.

Aufgabe der Erfindung ist es, ein Verfahren zur Herstellung von Enzym-Granulaten, insbesondere mit niedrigem Staubgehalt, zu schaffen, bei dem die Enzym-Granulate kontinuierlich oder chargenweise unter weitester Vermeidung von Temperaturungleichverteilungen im Herstellungsprozess und bei Erhöhung der Ausbeute an (relativer) Aktivität von Enzymen hergestellt werden können. Gleichzeitig soll die Kontrollierbarkeit der Granulation bei der Herstellung verbessert werden. Wichtige Aufgabe der vorliegenden Erfindung ist insbesondere, ein Granulationsverfahren zu schaffen, das eine kürzere Verweilzeit im Vergleich mit den bekannten Wirbelschichtverfahren unter sonst gleichen Bedingungen, wie Zusammensetzung des Enzymkonzentrats, Trocknungslufttemperaturen, mittlere Korngröße D50 der Granulate und Rundheit der Granulate ermöglicht. Diese Aufgabe wird erfindungsgemäß durch die kennzeichnenden Merkmale des Patentanspruches 1 gelöst, die ein darüber hinaus besonders schonendes Verfahren beschreiben.

Erfindungsgemäß erfolgt die Herstellung von Enzym-Granulaten durch eine Verknüpfung zwischen den thermischen Bedingungen in der Sprühzone und den Temperaturbedingungen im übrigen Bereich des Apparates mittels der in Anspruch 1 im kennzeichnenden Teil genannten Merkmale. Insbesondere können gegenüber den Verfahren aus dem Stand der Technik verringerte Materialverweilzeiten erreicht werden, was zu einer höheren relativen Enzymaktivität in den mittels des in Anspruch 1 genannten Verfahrens gewonnenen Enzym-Granulaten führt. Im erfindungsgemäßen Prozess wird dies dadurch erreicht, dass die Zuführung des erhitzten Prozessgases zur Trocknung hauptsächlich; d.h. insbesondere zu mehr als 80 %, vorzugsweise ausschließlich im Bedüsungsbereich erfolgt. Die sichere Zuführung von Teilchen in den Bedüsungsbereich hinein erfolgt insbesondere durch die spezielle geometrische Gestaltung des Apparates unter Nutzung der Schwerkraft, kann aber auch pneumatisch oder durch Kombination der geometrischen Gestaltung unter Nutzung der Schwerkraft und pneumatischer Zuführung geschehen.

Der Vorteil der erfindungsgemäßen Lösung nach Anspruch 1 besteht darin, dass die Herstellungsbedingungen an die herzustellenden Materialeigenschaften angepasst werden. Temperaturungleichverteilungen werden weitestgehend vermieden, wodurch auch eine Steigerung der Ausbeute an Enzym-Granulate; erreicht wird.

Mit der vorliegenden Erfindung gelingt es auch, ein Enzym-Granulat zur Verfügung zu stellen mit niedrigem Staubanteil und höherem (relativem) Anteil an aktivem Enzym als im Stand der Technik in Kombination mit einer mittleren Korngröße D50 von 60 (insbesondere 100) µm bis 2000 µm, guter Lagerstabilität, insbesondere einem kleinen Rundheitsfaktor, und/oder geringen Feuchtegehalt.

Die nach dem erfindungsgemäßen Verfahren in Anspruch 1 und insbesondere den Unteransprüchen erhältlichen Enzym-Granulate weisen diese vorteilhaften Eigenschaften auf. Diese können vorteilhaft für die Herstellung von allerlei interessanten Formulierungenverwendet werden, insbesondere durch zusetzen eines oder mehrerer geeigneter Trägermaterialien und/oder Verpackung in geeigneten Anwendungsformen.

Weitere vorteilhafte Ausgestaltungen sind in den Unteransprüchen (die hier durch Bezugnahme aufgenommen werden) beschrieben, sie werden weitgehend in der Beschreibung zusammen mit ihrer Wirkung erläutert.

Die nach dem erfindungsgemäßen Verfahren herstellbaren Enzym-Granulate sind hochkonzentriert und wasserlöslich oder wasserdispergierbar und haben eine mittlere Korngröße D50 von 60 bis 2000 µm und sind insbesondere weiter gekennzeichnet durch einen Staubgehalt von < 800, vorzugsweise kleiner 500 ppm nach dem Heubachtest bei einem Verhältnis aktiver Enzymgehalte zur Summe aus aktiven und inaktiven Enzymgehalten (relative Enzymaktivität) von 80 % oder größer, insbesondere 88 % oder mehr. Die Druckfestigkeit der herstellbaren Enzym-Granulate liegt vorteilhaft bei 10 MPa oder höher, in einer möglichen bevorzugten Ausführungsform der Erfindung bei 20 bis 50 MPa, und die Schüttdichte liegt bei 500 g/l oder mehr, in einer möglichen bevorzugten Ausführungsform bei 550 bis 850 g/l. Die Korngrößenverteilung, gekennzeichnet durch das Verhältnis d_{10/}d₉₀ (Definition: d₁₀ ist der Korndurchmesser, bei dem 10 % der Masse des Granulats kleiner sind als dieser Durchmesser, d₉₀ ist der Korndurchmesser, bei dem 90 % der Masse des Granulats kleiner sind als dieser Durchmesser), liegt insbesondere bei 0,4 oder höher. Die absolute Phytaseaktivität eines erfindungsgemäß vorteilhaft herstellbaren Enzym-Granulats (hier beinhaltend Phytase als Enzym) ist vorzugsweise gleich oder größer als 15 000 FTU/g. Dabei ist eine FTU die Enzymaktivität, welche 1 Micromol Phosphat pro Minute bei 37 °C unter Assay-Bedingungen (0,25 M Natriumacetat, pH-Wert von 5,5; 51 nM Natriumphytat) freisetzt.

Die Erfindung wird nachfolgend anhand einer bevorzugten Ausführungsform näher erläutert. In der dazugehörigen Zeichnungen ist schematisch eine Anlage zur Durchführung des erfindungsgemäßen Verfahrens dargestellt.

Die zur Trocknung der herzustellenden Enzym-Granulate erforderliche Menge an erwärmtem Prozessgas 10 (in der Regel erhitzte Luft) wird einer Zuluftkammer 17, mit rechteckigem Querschnitt 9 und begrenzenden Seitenwänden 5, zugeführt. In der Zuluftkammer 17 verteilt sich das Prozessgas 10 und tritt über Spaltöffnungen 1 in den Prozessraum 8 in Form von Gasstrahlen 2 ein. Der vorzugsweise horizontal in den Spalt 1 eintretende Prozessgasstrom wird durch das Umlenkteil 3 vorzugsweise nach oben in den Prozessraum 8 hinein umgelenkt und strömt als eine Art Freistrahl in den Apparat hinein. Des weiteren kann sich der Apparatequerschnitt optional in der Expansionszone 14 vergrößern, so dass sich die Geschwindigkeit der Prozessgasströmung nach oben hin stetig verringert. Das Gas verlässt den Apparat als Abgas 11 oberhalb der Expansionszone 14 über das Abluftteil 19, in das optional ein Entstaubungssystem (z.B. Filterpatronen oder Textilfilterelemente) integriert werden kann.

Im Prozessraum 8 befindet sich eine Menge an Partikeln, die durch den Prozessgasstrahl nach oben hin mitgerissen werden. Im oberen Bereich des Prozessraumes 8 sowie in der darüber befindlichen Expansionszone 14 nimmt die Gasgeschwindigkeit ab, so dass die aufwärts strömenden Teilchen seitlich aus dem Gasstrahl 23 heraustreten und in den Prozessraum 8 zurückfallen. Der Prozessraum 8 wird im unteren Bereich von geneigten Seitenwänden 29 begrenzt. Bedingt durch diese Seitenneigung werden die Teilchen unter Wirkung der Schwerkraft über die Rücklaufzone 24 in Richtung des Gaseintrittsspaltes 1 befördert, wo sie anschließend wieder vom Prozessgas in den Prozessraum 8 mitgerissen werden.

Durch diesen Mechanismus bildet sich eine sehr gleichförmige Feststoffzirkulation 15 bestehend aus einer Aufwärtsströmung und einem Rücklauf in Richtung des Prozessgaseintrittes. Dadurch liegt auch bei sehr geringen Mengen an Teilchen im Prozessraum 8 in der Kernzone oberhalb des Umlenkteiles 3 eine hohe Partikeldichte vor. In diesem Bereich werden ein oder mehrere Sprühdüsen 7 angeordnet, die gleichgerichtet zum Prozessgasstrahl nach oben sprühen und zum Einbringen der flüssigen Enzymformulierung dienen.

Durch die hohe Partikelbeladung in der Kernzone ergeben sich in der Bedüsungszone 22 sehr vorteilhafte Bedingungen für den Wärme- und Stoffübergang. Weiterhin wird erreicht, dass sich die Flüssigkeit weitestgehend an den Teilchen abscheidet und diese somit gleichmäßig an den Partikeloberflächen benetzt werden. Das gleichmäßige Benetzen bei gleichzeitiger hoher Feststoffzirkulation zwischen Bedüsungsbereich und Rücklaufzone 24 bewirkt, ,dass ein sehr gleichmäßiger Flüssigkeitsfilm gebildet wird. Durch den Trocknungsprozess verdampft die Flüssigkeit und verlässt mit dem Abgas 11 den Apparat. Der in der Formulierung enthaltene Feststoff verbleibt auf der Teilchenoberfläche. Dadurch wachsen die Granulate sehr gleichförmig und homogen, was zu einer sehr engen Korngrößenverteilung führt. Durch die in dem Prozessraum 8 ausgebildete kreisähnliche Feststoffströmung wird im Bereich der Sprühdüsen 7 und 6 ein Sprühtrocknungsbereich und daran anschließend ein Granulationsbereich ausgebildet.

Das Prozessgas kann einen Teil der Partikeln sowie Feingut und Stäube als feststoffbeladene Abluft 20 aus dem Prozessraum 8 austragen. Zur Abscheidung dieser Teilchen kann das im Abluftteil 19 optional integrierte Filtersystem oder dem Apparat nachgeschaltete Entstaubungsanlagen verwendet werden. Im Falle einer integrierten Entstaubungsanlage 25 können beispielsweise Druckluftimpulse 18 genutzt werden, um die zurückgehaltenen Partikeln als abgetrennter Feststoff 21 in den Prozessraum 8 zurückzuführen.

Im Vergleich zu Wirbelschichtapparaten mit integrierten Filteranlagen wird die Staubrückführung dadurch erleichtert, dass die aufwärts gerichtete Prozessgasströmung im wesentlichen örtlich begrenzt ist und somit die zurückzuführenden Teilchen außerhalb des Gasstrahles sicher absinken können. Durch die Sogwirkung in der Nähe des Gaseintrittsspaltes 1 wird dieser Mechanismus zusätzlich gefördert. Alternativ können von der Abluft abgeschiedene Teilchen oder anderweitig gewonnene enzymhaltige Teilchen (siehe unten) in den Prozessraum 8 zurückgeführt werden. Dazu können im unteren Bereich der geneigten Seitenwände 29 verschiedenartigste Zuführungen 26 angeordnet sein. Bedingt durch die hohe Geschwindigkeit des Prozessgasstrahls in der Nähe des Gaseintrittsspaltes 1 werden die feinen Partikeln angesaugt und der Bedüsungszone 22 zugeführt, wo diese mit Flüssigkeit benetzt werden und am Wachstumsprozess teilnehmen.

Optional eingebaute Leitbleche 16 stützen den Gasstrahl, verstärken den Sogeffekt und verbessern die Zuführung der Feststoffe in die Bedüsungszone 22 hinein. Eventuell auftretende Agglomerationseffekte werden minimiert, da im Bedüsungsbereich sehr hohe Strömungsgeschwindigkeiten und somit höhere Trennkräfte als in Wirbelschichten auftreten. Dadurch werden Teilchen separiert und wachsen zu sehr kugeligen Granulaten.

Das Strömungsprofil des Prozessgases im Prozessraum 8 bewirkt weiterhin, dass von der optional integrierten Filteranlage in den Prozessraum zurückgeführte Feinpartikel nicht in die Bedüsungszone 22 zurückfallen. Dadurch werden das Verkleben von Feinpartikeln und daraus folgende Agglomeratbildungsprozesse unterbunden.

Zur kontinuierlichen Prozessführung kann der Apparat mit optional unterschiedlichen Eintragsystemen 13 für Feststoffe ausgerüstet werden. Dadurch können beispielsweise Enzym-Partikel dem Prozess zugeführt werden, die z.B. durch Zerkleinerung von beispielsweise (zu großen) Granulaten gewonnen werden können oder/und aus zu kleinen Granulaten bestehen, oder aus einem oder mehreren Enzympartikeln oder enzymhaltigen Edukten in Form anderweitig gewonnener ausreichend feiner Stäube und/oder Pulver bestehen. Derartige Enzympartikel oder enzymhaltige Edukte (enzymhaltige Zwischenprodukte) können Produkte anderer Prozessstufen und Verfahren (z.B. Sprühtrocknung von Enzymlösungen) sein. Der Anteil dieser eingetragenen enzymhaltigen Zwischenprodukte beträgt insbesondere 1 Gew.-% oder mehr, in einer möglichen bevorzugten Ausführungsform der Erfindung 5 bis 20 Ges.-%. Dabei ist es auch möglich und kann vorteilhaft sein, dass die eingetragenen Enzympartikel durch eine separate Sprühtrocknung einer Enzymsuspension hergestellt werden. Dabei ist es auch möglich, in einer möglichen vorteilhaften Ausführungsform der Erfindung bereits von Anfang an Enzympartikel zuzuführen. Diese Teilchen dienen dann als Granulationskeime oder als Startfüllung zur Verkürzung der Inbetriebnahmezeit. Außerdem können hier Additive in fester Form in den Prozess eingeschleust werden, die in die Enzym-Granulate eingebettet werden sollen. In einer weiteren möglichen bevorzugten Ausführungsform können, vorzugsweise vor oder insbesondere gleichzeitig mit oder nach Schritt a., wie oben oder nachfolgend erwähnt, zu Beginn oder während des Granulationsprozesses anstelle von Enzympartikeln andere feinkörnige bis körnige partikuläre Materialien (bevorzugte Partikelgröße kleiner als 0,5 mm, vorzugsweise 0,1 bis 0,2 mm), vorzugsweise inerte (also in erster Linie enzymatisch nicht aktive) partikuläre Materialien, beispielsweise zur Einstellung der enzymatische Aktivität der Enzym-Granulate, etwa durch Einführung entsprechender inerter Kerne, wie inerter Salzkerne, als Keimmaterial zugeführt werden. Der Gewichtsanteil der inerten Kerne kann dabei beispielsweise zwischen 0 und 95 Gew.-% am fertigen Enzym-Granulat betragen.

Alternativ oder ergänzend zu dieser Ausführungsform können während des Trocknungs- und Granulationsprozesses oder während eines oder mehrerer Teile dieser Prozesse ein oder mehrere inerte Materialien, wie insbesondere Salze und/oder Bindemittel, nicht nur als Kern- oder Keimmaterial, sondern zur Verdünnung des oder der Enzyme oder insbesondere der (absoluten, also aktive und inaktive Enzymkomponenten beinhaltenden) Enzymaktivität in der Matrix der Enzym-Granulate (also verteilt innerhalb von Teilen oder der gesamten Matrix) zugeführt werden, was eine weitere besonders bevorzugte Ausführungsform der Erfindung darstellt. Dabei können das oder die inerten Materialien zugeführt werden als Feststoff, beispielsweise durch Eintragungssysteme für Feststoffe wie 13, innerhalb der Enzymlösung(en) [= flüssigen Enzymformulierung(en)] (gelöst und/oder in Suspension), und/oder insbesondere in einer oder mehreren (vorzugsweise wässrigen) von der Enzymlösung separaten Lösungen, Suspensionen oder Schmelzen, insbesondere in die Gasstrahlen 2, über Zuführungen 26 und/oder in erster Linie über Düsen beispielsweise in der Bedüsungszone 22. Im letzteren Fall kann die Lösung oder Suspension oder ferner Schmelze des oder der inerten Materialien (z.B. eines Salzes, wie eines anorganischen Salzes eines (beispielsweise Alkali-) Metallsalzes, wie Natriumsulfat oder Kochsalz, vorzugsweise in Gegenwart eines Bindemittels) über ein oder mehrere separate Düsen neben der oder den Düsen für die Verdüsung der Enzymlösung insbesondere im Bereich der Gasstrahlen 2 verdüst werden, oder es können vorteilhaft 3-oder Mehrstoffdüsen verwendet werden. In diesem Falle werden die Flüssigkeiten getrennt in die jeweiligen Düsenanteile gegeben und in einer günstigen Ausführungsform der Erfindung mit ebenfalls zugeführtem (vorzugsweise Druck) Gas, wie Druckluft, zerstäubt. Die Düse besitzt mit Vorteil eine Anzahl konzentrischer Rohre, über welche die Flüssigkeiten und die Düsenluft zugeführt werden. Beispielsweise kann eine erste Flüssigkeit über das Innenrohr, eine zweite Flüssigkeit über einen außen anschließenden koaxialen Ringspalt und das Gas zur Verdüsung über einen weiteren noch weiter außen gelegenen koaxialen Ringspalt erfolgen (Dreistoffdüse), oder eine erste Flüssigkeit wird über das Innenrohr, das Gas zur Verdüsung über einen außen davon als erster folgenden ersten koaxialen Ringspalt, eine zweite Flüssigkeit über einen außerhalb des letzteren gelegenen weiteren koaxialen Ringspalt und weiteres Gas zur Verdüsung über einen dritten außen gelegenen koaxialen Ringspalt verdüst (Vierstoffdüse).

Diese Zufuhr von inertem Material (als Keim im Kern, als Zusatz in der Matrix der Granulate oder beides) ermöglicht, bei hoher relativer Aktivität des verwendeten Enzymmaterials (geringe Inaktivierung) gewünschte absolute Aktivitäten (Aktivität je Gewichtsmenge Granulat) sehr präzise beliebig (d.h. zwischen geringfügig über 0 bis 100 % der maximal möglichen absoluten Aktivität) einzustellen, ohne dabei die übrigen Parameter des Enzym-Granulates, wie die Korngröße oder die Staubfreiheit, zu verändern. Sie kann im kontinuierlichen Betrieb oder im Batch-Betrieb chargenweise erfolgen. Der Anteil des Zusatzes an inertem Material kann 0 bis nahezu 100 %, beispielsweise von 0,1 bis 95 Gew.-%, bezogen auf den Feststoffanteil des Enzym-Granulats, betragen. Die Korngröße des inerten Materials kann, sofern es gelöst eingesetzt wird, beliebig sein, bei Einsatz als Feststoffpulver oder als Suspension liegt die Korngröße vorteilhaft bei 200 µm oder weniger, insbesondere bei 100 µm oder weniger.

Dargestellt ist damit auch die Verwendung von inerten Materialien in den vor- und nachstehend beschriebenen Verfahren zur Einstellung einer bestimmten absoluten Enzymaktivität der Enzym-Granulate (Enzymaktivität pro (Gewichts-)Menge Enzym-Granulat).

Weiterhin kann der Apparat mit Austragsorganen 4 versehen werden, um Partikel aus dem Prozessraum 8 entnehmen zu können. Das kann beispielsweise durch einen Überlauf oder durch ein volumetrisches Austragsorgan (z.B. eine Zellenradschleuse) oder auch durch einen Schwerkraftsicher (z.B. einen mit Sichtgas beaufschlagten Zick-Zack-Sichter oder einen Steigrohrsichter) erfolgen.

Optional können mechanische Aggregate 27 im Prozessraum 8, jedoch vorzugsweise im Bereich der Rücklaufzone 24 an den geneigten Wänden angebracht werden, um durch Zerkleinerung ausreichend Feinmaterial als Keime für den Granulatbildungsprozess zu erzeugen. Weiterhin kann die Rücklaufzone 24 optional zur Positionierung von Beheizungen oder anderen Wärmeübertragungseinrichtungen 28 genutzt werden. Beispielsweise kann die Apparatewand doppelwandig ausgeführt sein, um diese beispielsweise unter Nutzung von flüssigen oder gasförmigen Wärmeträgern zur Beheizung oder Kühlung zu verwenden. Alternativ könnten auch Mikrowellenheizer genutzt werden, um die Partikel in der Rücklaufzone 24 nachzutrocknen oder vorzuwärmen.

Im Prozessraum 8 oder in den darüberliegenden Apparateteilen, z.B. der Expansionszone 14 und dem Abluftteil 19, können optional Sprühdüsen 6 angeordnet werden, die vorzugsweise nach unten, aber auch teilweise nach oben sprühen. Hier kann ebenfalls die flüssige Enzym-Formulierung eingedüst werden, um beispielsweise durch Sprühtrocknung im Apparat Granulationskeime zu erzeugen. Alternativ können über einige der Sprüheinrichtungen 6 und 7 Additive oder andere Komponenten in flüssiger Form eingesprüht und somit in die Granulatstruktur homogen eingebettet werden. Wenn die Sprühdüsen 7 die heißgasbeaufschlagte Zuluftkammer 17 passieren, können optional die flüssigkeitsführenden Teile mit Isolationen oder unterschiedlichen Kühlsystemen 12 versehen werden, um Schädigungen an der flüssigen Formulierung zu unterbinden.

Zur Verminderung der Wasserempfindlichkeit und/oder zur Kontrolle der Wasserlöslichkeit der erfindungsgemäß hergestellten Enzym-Granulate können diese in einem nachfolgenden separaten Prozess durch Coating mit einem Schutzüberzug versehen werden.

Als weiterer Vorteil des erfindungsgemäßen Prozesses ist der sehr einfache Aufbau zu nennen, der eine hohe Betriebssicherheit und Störungsunempfindlichkeit mit sehr guter Reinigbarkeit verbindet. Somit werden verbesserte. Produktionsbedingungen insbesondere hinsichtlich der Hygieneanforderungen bei Produktwechsel bei biologischen Stoffen geschaffen.

### Beispiele :

Die Erfindung wird anhand der nachfolgenden konkreten Anwendungsbeispiele veranschaulicht, ohne dadurch in irgend einer Weise eingeschränkt zu werden.

### Beispiel 1: Herstellung von Enzym-Granulaten

Es wurde eine Enzymformulierung, die zusätzlich zur Enzymlösung einen Stabilisator sowie Binderkomponenten enthielt und eine Endkonzentration an Feststoffen von etwa 22 Massenprozent hatte, in einen Apparat eingesprüht, der durch den zuvor beschriebenen Aufbau gekennzeichnet ist. Der Prozessraum ist gekennzeichnet durch einen rechteckigen Querschnitt und hat oberhalb der geneigten Seitenwände eine Querschnittsfläche von 0,15x0,2=0,03m² und eine Höhe von etwa 1m. Die Zufuhr des auf etwa 140°C erwärmten Prozessluftstromes von etwa 180 kg/h erfolgte über 2 längs durch den Apparat verlaufende Gaszuführungsspalte. Die flüssige Formulierung wurde über eine druckluftbeaufschlagte vertikal nach oben sprühende Zweistoffdüse in den Prozessluftstrahl mit einem Massenstrom von etwa 50g/min eingesprüht. Im Prozessraum befanden sich etwa 500g an Enzym - Partikeln. Durch den Verdampfungsprozess kühlte sich die Prozessluft ab und verließ mit etwa 45°C den Apparat. Die Entstaubung der Abluft erfolgte durch einen dem Apparat nachgeschalteten Zyklon, und der abgeschiedene Feststoff wurde gravimetrisch in den Prozessraum in Spaltnähe als Keimmaterial zugeführt. Die Entnahme von Granulaten aus dem Prozessraum erfolgte stirnseitig unter Verwendung eines Siebes. Die im Sichter abgetrennten Feinanteile wurden pneumatisch in den Prozessraum zurückgeblasen. Das entnommene Granulat hat eine unverfestigte Schüttdichte von 800 g/l und folgende Korngrößenverteilung (Siebanalyse):

| | |
|---|---|
| > 400µm: | 0,8 Mass.-% |
| 315...400 µm: | 6,8 Mass.-% |
| 250 ... 315 µm: | 15,3 Mass.-% |
| 160...250 µm: | 42,3 Mass.-% |
| 100 ... 160 µm: | 24,9 Mass.-% |
| 0...100 µm: | 9,9 Mass.-% |

### Beispiel 2: Enzym-Granulate mit Phytase aus Aspergillus niger:

Kommerziell erhältliche Phytase (Natuphos 5000L, BASF, Ludwigshafen, Deutschland) wird diafiltriert mit demineralisiertem Wasser und einem Ultrafilter mit einer Porengröße, die das Enzym nicht passieren lässt, um Konservierungsmittel und Salze zu entfernen. Das Enzym wird anschließend ultrafiltriert, um ein hochkonzentriertes flüssiges Enzympräparat zu erhalten.

Zu 25 Gew.-% dieses flüssigen Enzympräparates mit einer Phytase-Aktivität von 24 000 FTU/g und einem Trockengehalt von 25 Gew.-% wird Polyvinylalkohol als Bindemittel hinzugefügt. Die übrigen 75 Gew.-% der Lösung werden sprühgetrocknet bei einer Lufteintrittstemperatur von 180 °C und einer Ablufttemperatur von 70 °C in dem in Beispiel 1 genannten Apparat.

Das sprühgetrocknete Enzympulver wird in einem staubdicht angedockten Behälter aufgefangen. Es resultiert ein Enzympulver mit einer Phytase-Aktivität von 90 000 FTU/g und 95 % Trockensubstanz. Der Behälter mit dem sprühgetrockneten Enzympulver wird mit einer staubdichten Kupplung an das Eintragsystem 13 angedockt. Das flüssige Enzympräparat wird mit einer Dosierpumpe durch eine Sprühdüse in den Prozessraum 8 gesprüht.

Flüssiges Enzympräparat und Enzympulver werden in einem Massenverhältnis von 4 : 1 zugeführt. Die Eintrittstemperatur liegt bei 120 °C, die Ablufttemperatur bei 60 °C. Es entsteht ein Phytase-Granulat mit den in Tabelle 1 gezeigten Eigenschaften. Der Gehalt an aktiver und an inaktiver Phytase wird bestimmt unter Verwendung der in EP 0 420 356 beschriebenen Vorgehensweise zur Charakterisierung von Aspergillus ficuum-Phytase - die Referenz wird hier diesbezüglich durch Bezugnahme aufgenommen.

**Tabelle 1: Eigenschaften der Phytase-Granulate nach Beispiel 2**

| Eigenschaft | Zahlenwert |
|---|---|
| Rundheitsfaktor | 1,4 |
| Restfeuchtigkeit | 5 % |
| Aktivitätsausbeute | 97 % |
| Gehalt an aktivem Enzym/Gesamtenzymgehalt | 95 % |
| Aktivität | 83 000 FTU/g |
| mittlere Korngröße D50 | 640 µm |
| Korngrößenverhältnis d₁₀/d₉₀ | 0,7 |
| Schüttdichte | 590 g/l |

### Beispiel 3: Verwendung von Salz-/Binderlösungen

Es wird eine Pilotanlage mit 4 Zuluftkammern und 4 Düsen verwendet. Als Enzym wird eine Protease eingesetzt. Anorganische Alkalimetallsalze und übliche Bindermittel finden für die Salz/Binderkomponenten Verwendung. Der Anteil der Komponenten wird in Gew.-% ("%") angegeben.
a) Reine Enzymlösung und Salz-Binderlösung werden jeweils unterschiedlichen Düsen zugeführt, die verdünnte Wassermenge pro Düse möglichst gleich eingestellt:

| | | Enzymlsg. (kalt) | Salz-Binder-Susp. (65°C) |
|---|---|---|---|
| Kammern | | 3 | 1 |
| Konzentration | % | 18 | 50 |
| Sprühmenge | kg/h | 22 | 12 |
| Wasser pro Düse | kg/h | 6,0 | 6 |
| Anteil im Produkt | % | 39,8 | 60,2 |
| | | | |
| | | | |
| Zulufttemperatur | °C | 125 | |
| Ablufttemperatur | °C | 55 | |

b) Enzymlösung und Salz-Binderlösung werden gemischt über alle Düsen zugeführt:

| | | Enzymanteil | Salz+Binderanteil |
|---|---|---|---|
| Kammern | | 4 | |
| Anteil in Lsg. | % | 10 | 24 |
| Sprühmenge | kg/h | 30 | |
| Wasser pro Düse | kg/h | 4,95 | |
| Anteil im Produkt | % | 29,4 | 70,6 |
| | | | |
| | | | |
| Zulufttemperatur | °C | 115 | |
| Ablufttemperatur | °C | 50 | |

c) Enzymlösung und Salz-Binderlösung werden getrennt über Dreistoffdüsen zugeführt:

| | | Enzymlsg.(kalt) | Salz-Binder-Susp. (65°C) |
|---|---|---|---|
| Kammern | | 4 | |
| Konzentration | % | 15 | 50 |
| Sprühmenge | kg/h | 15 | 20 |
| Wasser pro Düse | kg/h | 5,7 | |
| Anteil im Produkt | % | 18,4 | 81,6 |
| | | | |
| | | | |
| Zulufttemperatur | °C | 120 | |
| Ablufttemperatur | °C | 55 | |

d) Die Enzym-Binder-Lösung wird versprüht und Salzpulver wird in fester Form zugeführt

| | | Enzym-Binder-Lsg.(kalt) | Salzpulver <30µm |
|---|---|---|---|
| Kammern | | 4 | |
| Konzentration | % | 15 | 100 |
| Sprühmenge | kg/h | 20 | 25 |
| Wasser pro Düse | kg/h | 4,3 | |
| Anteil im Produkt | % | 10,7 | 89,3 |

### Zusammenfassend lässt sich folgendes feststellen:

Die Erfindung betrifft ein Verfahren zur Herstellung von Enzym-Granulaten. Aufgabe der Erfindung ist es, ein Verfahren zur Herstellung von Enzym-Granulaten zu schaffen, bei dem die Enzym-Granulate kontinuierlich oder chargenweise unter weitester Vermeidung von Temperaturungleichverteilungen im Herstellungsprozess und bei Erhöhung der Ausbeute an Aktivität von Enzymen hergestellt werden können. Gleichzeitig soll die Kontrollierbarkeit der Granulation bei der Herstellung verbessert werden. Mit dem Verfahren erhältliche Enzym-Granulate und deren Verwendung werden offengelegt.

Erfindungsgemäß erfolgt die Herstellung von Enzym-Granulaten durch eine Verknüpfung zwischen den thermischen Bedingungen in der Sprühzone und den Temperaturbedingungen im übrigen Bereich des Apparates. Im erfindungsgemäßen Prozess wird dies dadurch erreicht, dass die Zuführung des erhitzten Prozessgases zur Trocknung ausschließlich im Bedüsungsbereich erfolgt. Die sichere Zuführung von Teilchen in den Bedüsungsbereich hinein erfolgt durch die spezielle geometrische Gestaltung des Apparates unter Nutzung der Schwerkraft.

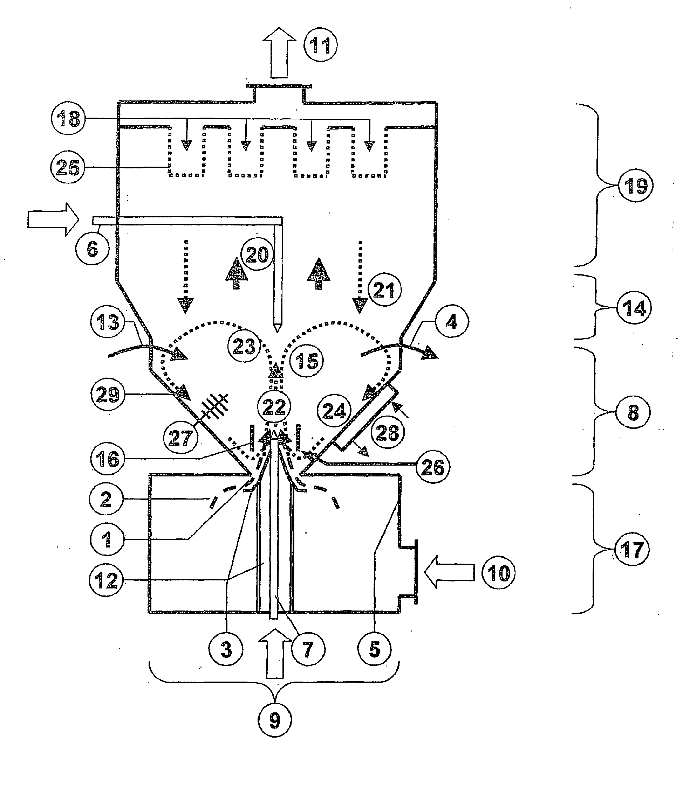

## Patentansprüche

1. Verfahren zur Herstellung von Enzym-Granulaten, **dadurch gekennzeichnet, dass**
a. ein oder mehrere flüssige Enzymformulierungen über Sprüheinrichtungen hauptsächlich in einen feststoffbeladenen ' Gasstrahl eingedüst werden,
b. die mit Flüssigkeit benetzten Materialteilchen im erwärmten Gasstrahl einem Trocknungs- und Granulationprozess unterzogen werden,
c. die Teilchen nach einer Verweilzeit vom Gasstrahl getrennt und in den Prozessraum zurückgeführt werde,
d. die Teilchen dem Gaseintrittsbereich zugeführt werden,
e. Feinpartikel, stäube und vom Prozessgas mitgerissene - Teilchen abgeschieden werden und dem Prozess als Keimmaterial nur den Granulatbildungsprozess wieder zugeführt werden,
f. durch Materialzuführung in den Gasstrahl eine in axialer Richtung des Reaktionsraumes liegende kreisähnliche feststoffströmung erzeugt wird.

2. Verfahren zur Herstellung von Enzym-Granulaten nach Anspruch 1, **dadurch gekennzeichnet, dass**
a. ein oder mehrere flüssige Enzymformulieruhgen über, Sprüheinrichtungen in einen feststoffbeladenen Gasstrahl eingedüst werden,
b. die mit Flüssigkeit benetzten Materialteilchen im erwärmten Gasstrahl einem Trocknungs- und Granulationsprozess unterzogen werden,
c. die Teilchen nach einer Verweilzeit vom Gasstrahl getrennt und in den Prozessraum zurückgeführt werden,
d. die Teilchen durch Schwerkraft über geneigte flächen dem Gaseintrittsbereich zugeführt werden,
e. Feinpartikel, Stäube und vom Prozessgas mitgerissene Teilchen abgeschieden werden und dem Prozess als Keimmaterial für den Granulatbildungsprozess wieder zugeführt werden,
f. durch Materialzuführung in den über die vorzugsweise rotationssymmetrischen oder langgestreckten Spaltöffnungen zugeführten Gasstrahl(en) eine in axialer Richtung des Reaktionsraumes liegende kreisähnliche Feststoffströmung erzeugt wird.

3. Verfahren nach einem der Ansprüche 1 oder '2, **dadurch gekennzeichnet, dass** die Enzym-Granulate über unterschiedliche Sichtvorrichtungen aus dem Prozessraum entnommen werden.

4. Verfahren nach einem der Ansprüche 1 bis 3, **dadurch gekennzeichnet, dass** die Enzym-Granulate über unterschiedliche volumetrische Austragsorgane aus dem Prozessraum entnommen werden.

5. verfahren nach einem oder mehren der Ansprüche 2 bis 4, **dadurch gekennzeichnet, dass** zu große oder zu kleine aus dem Prozess entnommene Enzym-Granulate vom Gutprodukt abgetrennt werden.

6. Verfahren nach einem oder mehren der Ansprüche 1 bis 5, **dadurch gekennzeichnet, dass** zu kleine aus dem Prozess entnommene Enzym-Granulate in den Prozessraum als Keimmaterial zurückgeführt werden.

7. Verfahren nach einem oder mehren der Ansprüche 1 bis 6, **dadurch gekennzeichnet, dass** zu große aus dem Prozess entnommene Enzym-Graaulate durch ein beliebiges Zerkleinerungsaggregat zerkleinert und in den Prozessraum als Keimmaterial zurückgeführt werden.

8. Verfahren nach einem oder mehren der Ansprüche 1, bis 7, **dadurch gekennzeichnet, dass** die in den Prozessraum zurückgeführten Enzym-Granulate thermisch nachbehandelt werden.

9. verfahren nach Anspruch 8, **dadurch gekennzeichnet, dass** die in den Prozessraum zurückgeführten Enzym-Granulate getrocknet oder vorgewärmt werden.

10. Verfahren nach einem oder mehren der Ansprüche 1 bis 9, **dadurch gekennzeichnet, dass** die in den Prozessraum zurückgeführten Enzym-Granulate zerkleinert werden.

11. Verfahren nach einem oder mehren der Ansprüche 1 bis 10, **dadurch gekennzeichnet, dass** Enzym-Granulate aus unterschiedlichen Zusätzen und mit unterschiedlichen Mischungsverhältnissen hergestellt werden.

12. Verfahren nach einem oder mehreren der Ansprüche 1 bis 11, **dadurch gekennzeichnet, dass** die Materialteilchen nach einer vorherigen Sprühtrocknung einem Granulationsprozess unterzogen werden.

13. Verfahren nach einem oder mehreren der Ansprüche 1 bis 12, **dadurch gekennzeichnet, dass** dem Granulationsprozess 1 Gew.-% oder mehr, vorzugsweise 5 bis 20 Gew.-% pulverförmigen Fertiggranulatprodukt, erhältlich nach einem der vorstehenden Ansprüche 1 bis 12, und/oder anderweitig gewonnene Enzympartikel und/oder ein oder mehrere enzymhaltige Zwischenprodukte ausgewählt aus enzymhaltige Pulvern und Stäuben zugeführt werden.

14. Verfahren nach einem oder mehreren der Ansprüche 1 bis 13, **dadurch gekennzeichnet, dass** die erhältlichen Enzym-Granulate in einem nachfolgenden Schritt durch Coating mit einem wasserschützenden Schutzfilm überzogen werden.

15. Verfahren nach einem oder mehreren der Ansprüchen 1 bis 14, **dadurch gekennzeichnet, dass** der Mittelwert der Verweilzeit der Enzyme im geheizten Prozessraum weniger als 1,5 Stunden, vorzugsweise weniger als 0,5 Stunden beträgt.

16. Verfahren nach einem der Ansprüche 1 oder 2, **dadurch gekennzeichnet, dass**, vorzugsweise vor oder insbesondere gleichzeitig mit oder nach Schritt a., wie in einem der Ansprüche 1 oder 2 erwähnt, oder während des Granulationsprozesses, als Keimmaterial feinkörnige bis körnige partikuläre Materialien, vorzugsweise inerte partikuläre Materialien, für den Trocknungs- und Granulationsprozeß zugeführt werden.

17. Verfahren nach einem der Ansprüche 1 oder 2 oder nach einem der Ansprüche 3 bis 15 oder 16, **dadurch gekennzeichnet, dass** während des Trocknungs- und Granulationsprozesses, oder während Teilen dieser Prozesse, ein oder mehrere inerte Materialien als Kern- oder Keimmaterial und/oder als Zusatz in der Enzym-Granulat-Matrix oder von Teilen davon zur Verdünnung des oder der Enzyme zugeführt werden.

18. Verfahren nach Anspruch 17, **dadurch gekennzeichnet, dass** das oder die inerten Materialien als Feststoff, innerhalb der Enzymlösung und/oder in einer oder mehreren von der Enzymlösung separaten Lösungen, Suspensionen oder Schmelzen zugeführt werden.

19. Verfahren nach einem der Ansprüche 17 oder 18, **dadurch gekennzeichnet, dass** eine oder mehrere Lösungen und/oder Suspensionen des oder der inerten Materialien über ein oder mehrere separate Düsen neben der oder den Düsen für die Verdüsung der flüssigen Enzymformulierung während des Trocknungs- und Granulationsprozesses, oder während Teilen davon, verdüst werden.

20. Verfahren nach einem der Ansprüche 17 bis 19, **dadurch gekennzeichnet, dass** eine oder mehreren Mehrstoffdüsen und ein Gas zur Zerstäubung einer oder mehrerer Lösungen oder Suspensionen eines oder mehrerer inerter Materialien verwendet werden.

## Claims

1. Method for producing enzyme granulates, **characterised in that**
a. one or more liquid enzyme formulations are injected mainly into a solids-bearing gas stream by spraying devices,
b. the material particles wetted with liquid in the heated gas stream undergo a drying and granulation process,
c. the particles are separated from the gas stream after a residence time and fed back into the processing space,
d. the particles are supplied to the gas inlet region,
e. fine particles, dust, and particles entrained by the processing gas are separated and fed back into the process as nucleus material for the granulate formation process,
f. a circle-like solids flow in an axial direction of the reaction space is generated by supplying material into the gas stream.

2. Method for producing enzyme granulates according to claim 1, **characterised in that**
a. one or more liquid enzyme formulations are injected into a solids-bearing gas stream by spraying devices,
b. the material particles wetted with liquid in the heated gas stream undergo a drying and granulation process,
c. the particles are separated from the gas stream after the residence time and fed back into the processing space,
d. the particles are supplied to the gas inlet region via inclined surfaces by the effect of gravity,
e. the fine particles, dust, and particles entrained by the processing gas are separated and fed back into the process as nucleus material for the granulate formation process,
f. a circle-like solids flow in an axial direction of the reaction space is generated by supplying material into the gas stream(s) supplied over the preferably rotationally symmetric or elongate gap openings.

3. Method according to either claim 1 or claim 2, **characterised in that** the enzyme granulates are removed from the processing space by different sifting devices.

4. Method according to any one of claims 1 to 3, **characterised in that** the enzyme granulates are removed from the processing space by different volumetric discharge elements.

5. Method according to one or more of claims 2 to 4, **characterised in that** enzyme granulates which are too large or too small and are removed from the process are separated from the material product.

6. Method according to one or more of claims 1 to 5, **characterised in that** enzyme granulates which are too small and are removed from the process are fed back into the processing space as the nucleus material.

7. Method according to one or more of claims 1 to 6, **characterised in that** enzyme granulates which are too large and are removed from the process are reduced in size by any desired size-reduction aggregate and fed back into the processing space as nucleus material.

8. Method according to one or more of claims 1 to 7, **characterised in that** the enzyme granulates fed back into the processing space are subsequently heat treated.

9. Method according to claim 8, **characterised in that** the enzyme granulates fed back into the processing space are dried or preheated.

10. Method according to one or more of claims 1 to 9, **characterised in that** the enzyme granulates fed back into the processing space are comminuted.

11. Method according to one or more of claims 1 to 10, **characterised in that** enzyme granulates made from different additives and having different mixture ratios are produced.

12. Method according to one or more of claims 1 to 11, **characterised in that** the material particles undergo a granulation process after previous spray drying.

13. Method according to one or more of claims 1 to 12, **characterised in that** 1 % by weight or more, preferably 5 to 20 % by weight, of pulverulent solid granulate product, obtained according to any one of the preceding claims 1 to 12, and/or enzyme particles created in another way, and/or one or more enzyme-containing intermediate products selected from enzyme-containing powders and dust are supplied to the granulation process.

14. Method according to one or more of claims 1 to 13, **characterised in that** the resulting enzyme granulates are covered with a water-resistant protective film by coating in a subsequent step.

15. Method according to one or more of claims 1 to 14, **characterised in that** the average value of the residence time of the enzymes in the heated processing space is less than 1.5 hours, preferably less than 0.5 hours.

16. Method according to either claim 1 or claim 2, **characterised in that** preferably before or in particular simultaneously with step a, as mentioned in either claim 1 or claim 2, or during the granulation process, fine-grained to granular particulate materials, preferably inert particulate materials, are supplied as nucleus material for the drying and granulation process.

17. Method according to either claim 1 or claim 2, or according to any one of claims 3 to 15 or 16, **characterised in that** during the drying and granulation process, or during parts of these processes, one or more inert materials are supplied as core or nucleus material and/or as an additive in the enzyme-granulate matrix or in parts thereof in order to dilute the enzyme(s).

18. Method according to claim 17, **characterised in that** the inert material(s) are supplied as solid material within the enzyme solution and/or in one or more solutions, suspensions or melts separate from the enzyme solution.

19. Method according to either claim 17 or claim 18, **characterised in that** one or more solutions and/or suspensions of the inert material(s) are sprayed by one or more separate nozzles in addition to the nozzle(s) for spraying the liquid enzyme formulation during the drying and granulation process, or during parts of these processes.

20. Method according to any one of claims 17 to 19, **characterised in that** one or more multi-material nozzles and a gas for atomising one or more solutions or suspensions of one or more inert materials are used.

## Revendications

1. Procédé de fabrication de granulés d'enzymes, **caractérisé en ce que**
a. une ou plusieurs formulations d'enzymes liquides sont, au moyen de dispositifs de pulvérisation, injectées principalement dans un jet de gaz chargé de matière solide,
b. les particules de matériau imprégnées de liquide sont soumises dans le jet de gaz chauffé à un processus de séchage et de granulation,
c. après un temps de séjour, les particules sont séparées du jet de gaz et ramenées dans la chambre de traitement,
d. les particules sont apportées à la zone d'entrée de gaz,
e. les particules fines, les poussières et les particules entraînées par le gaz de traitement sont isolées et rapportées au processus comme matériau de germination pour le processus de formation de granulés,
f. par apport de matériau dans le jet de gaz, on produit un flux de matière solide quasi circulaire situé dans la direction axiale de la chambre de réaction.

2. Procédé de fabrication de granulés d'enzymes selon la revendication 1, **caractérisé en ce que**
a. une ou plusieurs formulations d'enzymes liquides sont, au moyen de dispositifs de pulvérisation, injectées dans un jet de gaz chargé de matière solide,
b. les particules de matériau imprégnées de liquide sont soumises dans le jet de gaz chauffé à un processus de séchage et de granulation,
c. après un temps de séjour, les particules sont séparées du jet de gaz et ramenées dans la chambre de traitement,
d. les particules sont apportées à la zone d'entrée de gaz par la force de gravité, via des surfaces inclinées,
e. les particules fines, les poussières et les particules entraînées par le gaz de traitement sont isolées et rapportées au processus comme matériau de germination pour le processus de formation de granulés,
f. par apport de matériau dans le/les jets de gaz apportés par l'intermédiaire des ouvertures interstitielles de préférence oblongues ou à symétrie de révolution, on produit un flux de matière solide quasi circulaire situé dans la direction axiale de la chambre de réaction.

3. Procédé selon la revendication 1 ou 2, **caractérisé en ce que** les granulés d'enzymes sont prélevés de la chambre de traitement au moyen de différents dispositifs de triage.

4. Procédé selon l'une des revendications 1 à 3, **caractérisé en ce que** les granulés d'enzymes sont prélevés de la chambre de traitement au moyen de différents organes d'évacuation volumétriques.

5. Procédé selon l'une ou plusieurs des revendications 2 à 4, **caractérisé en ce que** les granulés d'enzymes prélevés du processus qui sont de taille excessive ou insuffisante sont séparés du produit final.

6. Procédé selon l'une ou plusieurs des revendications 1 à 5, **caractérisé en ce que** les granulés d'enzymes prélevés du processus qui sont de taille insuffisante sont ramenés dans la chambre de traitement comme matériau de germination.

7. Procédé selon l'une ou plusieurs des revendications 1 à 6, **caractérisé en ce que** les granulés d'enzymes prélevés du processus qui sont de taille excessive sont fragmentés par un organe de fragmentation quelconque et ramenés dans la chambre de traitement comme matériau de germination.

8. Procédé selon l'une ou plusieurs des revendications 1 à 7, **caractérisé en ce que** les granulés d'enzymes ramenés dans la chambre de traitement sont soumis à un retraitement thermique.

9. Procédé selon la revendication 8, **caractérisé en ce que** les granulés d'enzymes ramenés dans la chambre de traitement sont séchés ou préchauffés.

10. Procédé selon l'une ou plusieurs des revendications 1 à 9, **caractérisé en ce que** les granulés d'enzymes ramenés dans la chambre de traitement sont fragmentés.

11. Procédé selon l'une ou plusieurs des revendications 1 à 10, **caractérisé en ce qu'**on fabrique des granulés d'enzymes à partir de différents additifs et avec différentes proportions de mélanges.

12. Procédé selon l'une ou plusieurs des revendications 1 à 11, **caractérisé en ce que** les particules de matériau sont soumises à un processus de granulation après un séchage préalable par pulvérisation.

13. Procédé selon l'une ou plusieurs des revendications 1 à 12, **caractérisé en ce qu'**on apporte au processus de granulation 1 % en poids ou plus, de préférence 5 à 20 % en poids, de produit granulé fini en poudre, pouvant être obtenu selon l'une des revendications précédentes 1 à 12, et/ou des particules d'enzymes obtenues d'une autre manière et/ou un ou plusieurs produits intermédiaires contenant des enzymes, sélectionnés parmi des poudres et poussières contenant des enzymes.

14. Procédé selon l'une ou plusieurs des revendications 1 à 13, **caractérisé en ce que** les granulés d'enzymes pouvant être obtenus sont, au cours d'une étape suivante, revêtus par enrobage d'un film protecteur résistant à l'eau.

15. Procédé selon l'une ou plusieurs des revendications 1 à 14, **caractérisé en ce que** la valeur moyenne du temps de séjour des enzymes dans la chambre de traitement chauffée est inférieure à 1,5 heure, de préférence est inférieure à 0,5 heure.

16. Procédé selon la revendication 1 ou 2, **caractérisé en ce que**, de préférence avant ou notamment en même temps que ou après l'étape a. telle que mentionnée dans la revendication 1 ou 2, ou pendant le processus de granulation, on apporte comme matériau de germination pour le processus de séchage et de granulation des matériaux particulaires à grains fins à granuleux, de préférence des matériaux particulaires inertes.

17. Procédé selon la revendication 1 ou 2 ou selon l'une des revendications 3 à 15 ou 16, **caractérisé en ce que**, pendant le processus de séchage et de granulation, ou pendant des parties de ces processus, on apporte un ou plusieurs matériaux inertes comme matériau de noyau ou de germination et/ou comme additif dans la matrice de granulé d'enzyme ou des parties de celle-ci, pour diluer le ou les enzymes.

18. Procédé selon la revendication 17, **caractérisé en ce que** le ou les matériaux inertes sont apportés sous forme de matière solide, à l'intérieur de la solution d'enzyme et/ou dans une ou plusieurs solutions, suspensions ou masses fondues séparées de la solution d'enzyme.

19. Procédé selon la revendication 17 ou 18, **caractérisé en ce qu'**une ou plusieurs solutions et/ou suspensions du ou des matériaux inertes sont, pendant le processus de séchage et de granulation, ou pendant des parties de ce processus, pulvérisées au moyen d'une ou plusieurs buses séparées en plus de la ou des buses pour la pulvérisation de la formulation d'enzyme liquide.

20. Procédé selon l'une des revendications 17 à 19, **caractérisé en ce qu'**on utilise une ou plusieurs buses multi-matériaux et un gaz pour pulvériser une ou plusieurs solutions ou suspensions d'un ou plusieurs matériaux inertes.
